# EUROPÄISCHE PATENTSCHRIFT

(11) **EP 1 960 015 B1**
(45) Veröffentlichungstag und Bekanntmachung des Hinweises auf die Patenterteilung: **17.04.2013**
(21) Anmeldenummer: 06818960.4
(22) Anmeldetag: 01.12.2006
(51) Int. Cl.: A61M 1/14, A61M 1/34, A61M 1/16, A61M 1/36

(54) **ANORDNUNG AUS MEDIZINISCHEN BEHANDLUNGSEINHEITEN UND PERIPHERIEGERATEN**
ARRANGEMENT OF MEDICAL TREATMENT UNITS AND PERIPHERAL APPLIANCES
ENSEMBLE D'UNITES DE TRAITEMENT MEDICAL ET APPAREILS PERIPHERIQUES

(30) Priorität: 10.12.2005 DE 102005059131
(43) Veröffentlichungstag der Anmeldung: 27.08.2008
(73) Patentinhaber: Fresenius Medical Care Deutschland GmbH, 61352 Bad Homburg v.d.H. (DE)
(72) Erfinder: NIKOLIC, Dejan, 60599 Frankfurt am Main (DE); KÖNIG, Christoph, 65207 Wiesbaden/Auringen (DE)
(74) Vertreter: Oppermann, Frank
(86) Internationale Anmeldenummer: PCT/EP2006/011571
(87) Internationale Veröffentlichungsnummer: WO 2007/065609

(56) Entgegenhaltungen:
- WO-A-02/078775
- DE-A1-102004 011 264
- US-B1- 6 332 094

## Beschreibung

Die Erfindung betrifft eine Anordnung aus einer Mehrzahl von medizinischen Behandlungseinheiten und einer Mehrzahl von Peripheriegeräten, wobei jeweils eine Behandlungseinheit und ein Peripheriegerät einem Patienten zugeordnet werden.

In der Medizintechnik finden zur Behandlung von Patienten unterschiedliche Behandlttngseinheiten Verwendung, an denen die Patienten bzw. die an den Patienten angeschlossen werden. Die Verbindung zwischen Patient und Maschine erfolgt im Allgemeinen über Schläuche oder Leitungen. Zu den bekannten Behandlungseinheiten zählen beispielsweise Dialysemaschinen mit einem extrakorporalen Blutkreislauf.

Es ist bekannt, medizinische Behandlungseinheiten zusammen mit einem oder mehreren Peripheriegeräten zu betreiben. Derartige Satelliten dienen beispielsweise dazu, während der Behandlung den Patienten zu überwachen, wobei die ermittelten Daten an die Behandlungseinheit übertragen werden.

Während zwischen der Behandlungseinheit und dem Patienten über Schläuche oder Leitungen eine feste Verbindung besteht, kann die Datenübertragung zwischen dem Peripheriegerät und der Behandlungseinheit drahtlos erfolgen, beispielsweise per Funk- oder Lichtsignale. Folglich kann das Bedienungspersonal anhand der festen Verbindung zwar sofort die Zuordnung zwischen Behandlungseinheit und Patient erkennen, nicht aber die Zuordnung zwischen Peripheriegerät und Behandlungseinheit,

Aus der WO 02/078775 A2 ist eine Anordnung aus einer Mehrzahl von medizinischen Behandlungseinheiten und einer Mehrzahl von Peripherigeräten bekannt, wobei die medizinischen Behandlungseinheiten und die Peripheriegeräte jeweils einem Patienten zuzuordnen sind. Die Peripheriegeräte verfügen über Mittel zum Senden von Steuersignalen und Patientensignalen, während die Behandlungseinheiten über Mittel zum Empfangen von Steuersignalen und Patientensignalen von einem Peripheriegerät verfügen.

Aus der DE 10 2004 011 264 A1 ist eine Dialysestation bekannt, die mehrere Patientenplätze aufweist, die jeweils mit einem Dialysegerät und einem Bildschirm versehen sind. Die Bildschirme der Patientenplätze sind in ein Netzwerk eingebunden, das über einen Server verfügt. An einem Arbeitsplatz kann der Arzt den Verlauf der Behandlungen verfolgen, die mit den einzelnen Dialysegeräten der Dialysestation durchgeführt werden.

Wenn eine Mehrzahl von Behandlungseinheiten und Peripheriegeräten zusammen in einem Behandlungsraum betrieben werden, ist es erforderlich, jeweils eine Behandlungseinheit und ein Peripheriegerät einem Patienten zuzuordnen und zur Datenübertragung eine Verbindung von dem jeweiligen Peripheriegerät zu der jeweiligen Behandlungseinheit herzustellen. Eine falsche Zuordnung von Peripheriegerät und Behandlungseinheit hätte zur Folge, dass die Daten des einen Patienten auf die Behandlungseinheit des anderen Patienten übertragen werden.

Da eine falsche Datenübertragung im Extremfall zu lebensbedrohlichen Komplikationen während der Behandlung führen kann, ist sicherzustellen, dass eine richtige Zuordnung zwischen dem Peripheriegerät und dem Patienten einerseits und dem Peripheriegerät und der Behandlungseinheit andererseits getroffen wird. Dies ist bei den bekannten Peripheriegeräten und Behandlungseinheiten besonders problematisch, da bei einer kabellosen Verbindung die Zuordnung zwischen Peripheriegerät und Behandlungseinheit vom Personal nicht erkannt werden kann.

Wenn mehrere Geräte gleichzeitig betrieben werden, die miteinander kommunizieren, finden im Allgemeinen sogenannte Identifikations-(ID)-Signale Verwendung, mit denen erkannt werden kann, ob die Signale des einen oder anderen Satelliten empfangen werden. Die US 6,332,094 B1 beispielsweise beschreibt einen Pulsmesser, der die Pulssignale zusammen mit einem Identifikationssignal an einen Empfänger drahtlos überträgt.

Der Erfindung liegt die Aufgabe zugrunde, die Sicherheit und Flexibilität beim Betrieb einer Mehrzahl von medizinischen Behandlungseinheiten und Peripheriegeräten in einem gemeinsamen Behandlungsraum zu erhöhen.

Die Lösung dieser Aufgabe erfolgt erfindungsgemäß mit den Merkmalen des Patentanspruchs 1. Bevorzugte Ausführungsformen sind Gegenstand der Unteransprüche.

Bei der erfindungsgemäßen Anordnung, die über eine Mehrzahl von medizinischen Behandlungseinheiten und Peripheriegeräten verfügt, von denen jeweils eine Behandlungseinheit und ein Peripheriegerät einem Patienten zugeordnet werden, wird die Zuordnung des Peripheriegerätes zu dem Patienten einerseits und die Zuordnung des Peripheriegerätes zu der Behandlungseinheit andererseits überwacht. Hierfür verfügen die Peripheriegeräte über Mittel zur Überwachung der Zuordnung eines Peripheriegerätes zu einem Patienten und die Behandlungseinheiten über Mittel zur Überwachung der Zuordnung eines Peripheriegerätes zu der Behandlungseinheit. Darüber hinaus verfügen die Peripheriegeräte über Mittel zum Anmelden des Peripheriegerätes bei einer Behandlungseinheit und die Behandlungseinheiten über Mittel zur Eingabe einer Bestätigung einer erfolgreichen Zuordnung von einem Peripheriegerät zu der Behandlungseinheit.

Zunächst wird überprüft, ob ein Peripheriegerät einem Patienten zugeordnet ist. Wenn ein Peripheriegerät einem Patienten zugeordnet ist, sendet das Peripheriegerät ein Anmeldesignal an die dem Patienten zugeordnete Behandlungseinheit.

Die erfindungsgemäße Anordnung erfordert eine Bestätigung der erfolgreichen Zuordnung von Peripheriegerät und Behandlungseinheit. Nur wenn die Zuordnung bestätigt worden ist, kann die Datenübertragung zwischen dem Peripheriegerät und der Behandlungseinheit erfolgen.

Für die Anmeldung der Peripheriegeräte bei den Behandlungseinheiten verfügen die erfindungsgemäßen Peripheriegeräte über Mittel zum Senden von Steuersignalen an die Behandlungseinheiten und die Behandlungseinheiten über Mittel zum Empfangen von Steuersignalen von den Peripheriegeräten. Für die Datenübertragung verfügen die Peripheriegeräte über Mittel zum Senden von Patientensignalen an die Behandlungseinheiten und die Behandlungseinheiten über Mittel zum Empfangen von Patientensignalen von den Peripheriegeräten. Die Datenübertragung zwischen Peripheriegerät und Behandlungseinheit kann grundsätzlich nur unidirektional aber auch bidirektional erfolgen.

Die erfindungsgemäße Anordnung zwingt das Behandlungspersonal dazu, bei einer erfolgreichen Zuordnung eines Peripheriegerätes zu einem Patienten die dann zu erfolgende Zuordnung des Peripheriegerätes zu der dem Patienten zugeordneten Behandlungseinheit zu überprüfen. Dadurch wird ausgeschlossen, dass eine fehlerhafte Datenübertragung erfolgt.

Bei einer bevorzugten Ausführungsform weisen die Mittel zur Überwachung der Zuordnung eines Peripheriegerätes zu einer Behandlungseinheit ein Zeitglied mit einer vorgegebenen Zeitkonstante auf, das nach dem Empfang des Anmeldesignals gestartet wird. Die Datenübertragung von dem Peripheriegerät auf die Behandlungseinheit wird nur dann freigegeben, wenn das Bedienungspersonal innerhalb der vorgegebenen Zeitkonstante, die beispielsweise zwischen 1 und 60 Sekunden, aber auch zwischen einer oder mehreren Minuten liegen kann, die korrekte Zuordnung von Peripheriegerät und Behandlungseinheit bestätigt hat. Dadurch wird das Bedienungspersonal gezwungen, die Geräteanmeldung sofort an der Behandlungseinheit zu überprüfen, so dass nicht die Gefahr besteht, dass das Bedienungspersonal aufgrund anderer Aufgaben von der Bestätigung der Zuordnung abgelenkt wird.

Bei einer weiteren bevorzugten Ausführungsform sind die Mittel zur Überwachung der Zuordnung eines Peripheriegerätes zu einer Behandlungseinheit derart ausgebildet, dass die Datenübertragung von dem Peripheriegerät zu der Behandlungseinheit dann nicht freigegeben wird, wenn innerhalb der Zeitkonstanten Anmeldesignale von mehreren Peripheriegeräten empfangen worden sind. Dadurch wird sichergestellt, dass nur ein Peripheriegerät der Behandlungseinheit zugeordnet werden kann.

Bei einer weiteren bevorzugten Ausführungsform erfolgt die Zuordnung eines Peripheriegerätes zu einer Behandlungseinheit unter Verwendung von Identifikationssignalen. Bei einer ersten alternativen Ausführungsform sind die entsprechenden Identifikationssignale der einzelnen Peripheriegeräte in den Peripheriegeräten und den Identifikationssignalen der Peripheriegeräte zugeordnete Referenzsignale für die Erkennung der jeweiligen Peripheriegeräte in den Behandlungseinheiten nicht flüchtig gespeichert. Hierfür verfügen die Peripheriegeräte und Behandlungseinheiten über entsprechende Speicher. Bei einer weiteren besonders bevorzugten Ausführungsform werden das Identifikationssignal des Peripheriegerätes nach einer erfolgreichen Zuordnung von Peripheriegerät und Patient zusammen mit dem Anmeldesignal an die Behandlungseinheit gesendet, wobei die Behandlungseinheit nach der Bestätigung der erfolgreichen Anmeldung durch das Bedienungspersonal wiederum ein eigenes Identifikationssignal an das angemeldete Peripheriegerät sendet. Während der Datenübertragung werden dann beide Identifikationssignale verwendet.

Die Mittel zur Überwachung der Zuordnung eines Peripheriegerätes zu dem Patienten können Mittel zur manuellen Eingabe aufweisen. Wenn das Bedienungspersonal das Peripheriegerät dem Patienten zugeordnet hat, beispielsweise das Peripheriegerät an den Patienten angebracht hat, bestätigt das Bedienungspersonal die erfolgreiche Zuordnung durch eine manuelle Eingabe. Damit ist die Zuordnung zwischen Peripheriegerät und Patienten erfolgt.

Bei einer alternativen Ausführungsform kann die Zuordnung des Peripheriegerätes zu dem Patienten auch automatisch erfolgen. Hierzu verfügen die Mittel zur Überwachung der Zuordnung vorzugsweise über Mittel zur automatischen Erkennung eines dem Patient zugeordneten Peripheriegeräts, beispielsweise einen Nährungssensor oder dergleichen. Es ist aber auch möglich, dass auf eine erfolgreiche Zuordnung zwischen Peripheriegerät und Patient dann geschlossen wird, wenn beispielsweise ein Funktionstest des Peripheriegerätes erfolgreich war.

Das Problem einer falschen Zuordnung tritt nicht nur bei einer nicht eindeutigen Zuordnung von Peripheriegerät und Behandlungseinheit auf, sondern auch bei einer nicht eindeutigen Maschinenzuordnung. Die Datenübertragung wird daher unterbunden, wenn sich beispielsweise zwei Behandlungseinheiten mit einem Peripheriegerät verbinden wollen.

Die erfindungsgemäße Anordnung schafft ein großes Maß an Flexibilität bei der Gerätezuordnung, da die einzelnen Peripheriegeräte untereinander ausgetauscht und den einzelnen Behandlungseinheiten zugeordnet werden können, ohne dass die Gefahr einer falschen Datenübertragung besteht.

Im Folgenden wird ein Ausführungsbeispiel der Erfindung unter Bezugnahme auf die Zeichnungen näher erläutert:
Es zeigen:
   - Figur 1: eine Prinzipdarstellung einer korrekten Zuordnung zwischen zwei Peripheriegeräten und zwei Behandlungseinheiten,
   - Figur 2: die Anordnung von Figur 1, wobei die Peripheriegeräte den falschen Behandlungseinheiten zugeordnet sind, und
   - Figur 3: die wesentlichen Komponenten einer Behandlungseinheit und eines Peripheriegerätes.

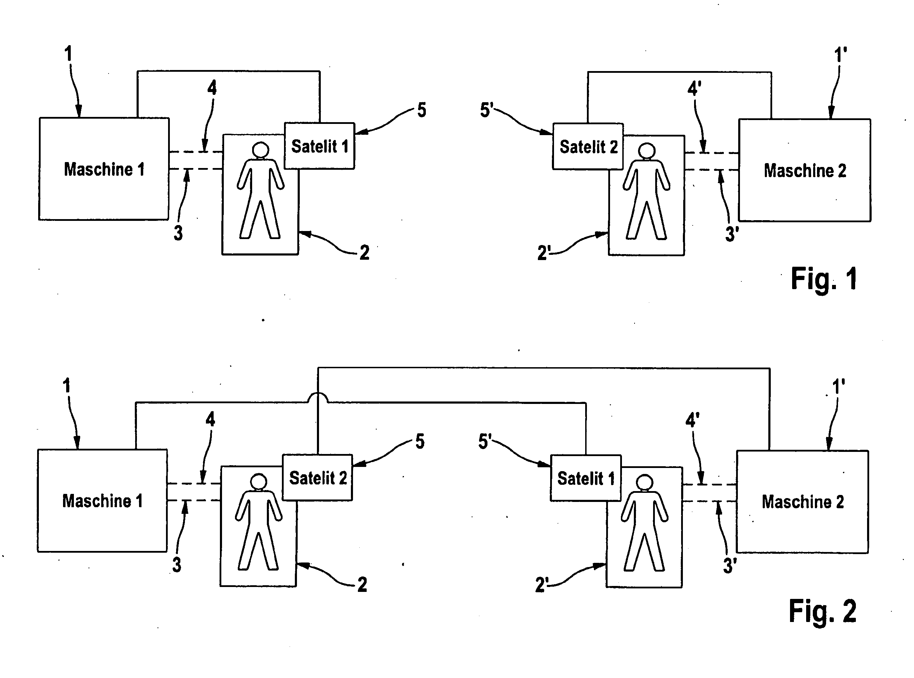

Die Figur 1 zeigt zwei medizinische Behandlungseinheiten 1, 1', beispielsweise extrakorporale Blutbehandlungsvorrichtungen mit einem extrakorporalen Blutkreislauf. An jeder Behandlungseinheit, 1, 1' ist ein Patient 2, 2' angeschlossen. Für den Fall einer extrakorporalen Blutbehandlung ist der Patient 2, 2' mit der Blutbehandlungsvorrichtung 1, 1' über eine venöse und arterielle Schlauchleitung 3, 4; 3', 4' verbunden. Damit ist eine feste Zuordnung zwischen dem Patienten und der Behandlungseinheit getroffen. Darüber hinaus ist den beiden Patienten 2, 2' jeweils ein Peripheriegerät 5, 5', beispielsweise ein Blutdruckmonitor zugeordnet.

Die Peripheriegeräte 5, 5' überwachen die Körperfunktionen des Patienten, beispielsweise den Blutdruck, und übertragen die Daten an die zugehörige Behandlungseinheit, an die der Patient angeschlossen ist.

Die Figur 1 zeigt die richtige Zuordnung von Peripheriegerät und Patient einerseits und Peripheriegerät und Behandlungseinheit andererseits, während Figur 2 eine falsche Zuordnung zwischen Peripheriegerät und Behandlungseinheit zeigt. Bei einer falschen Zuordnung empfängt die Behandlungseinheit 1 die Daten des Peripheriegerätes 5', während die Behandlungseinheit 1' die Daten des Peripheriegerätes 5 empfängt.

Die Kommunikation zwischen den Peripheriegeräten und den Behandlungseinheiten 1, 5; 1', 5' umfasst sowohl Steuersignale, die der Anmeldung (Anmeldesignal) und Identifikation (Identifikationssignal ID) der Geräte dienen, als auch Patientensignale, die der eigentlichen Datenübertragung dienen, beispielsweise des gemessenen Blutdrucks von dem Blutdruckmonitor zu der extrakorporalen Blutbehandlungsvorrichtung.

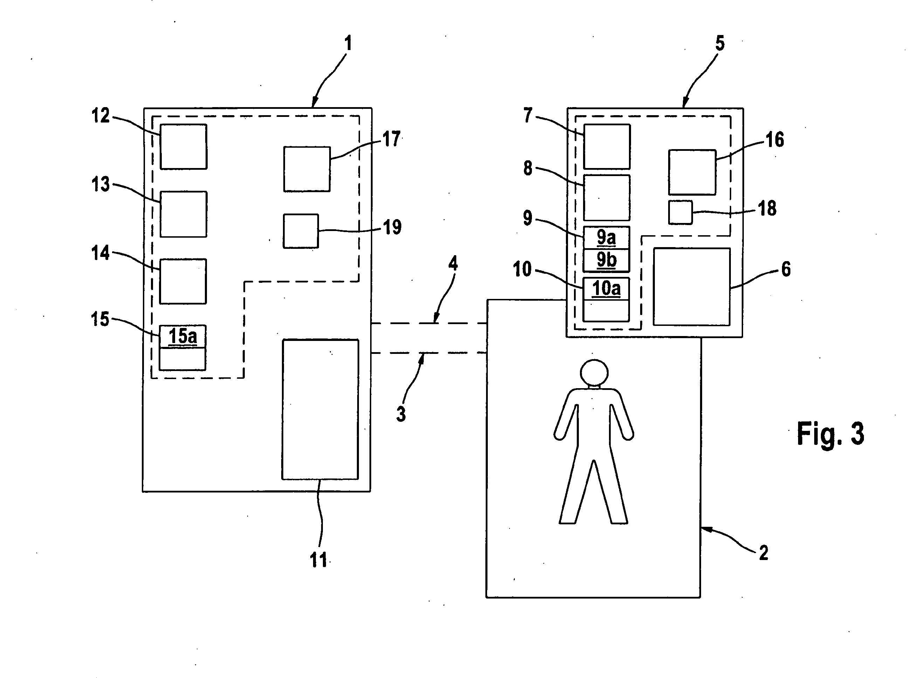

Die erfindungsgemäße Anordnung umfasst mindestens zwei medizinische Behandlungseinheiten und Peripheriegeräte. Im Folgenden werden die einzelnen Komponenten einer Behandlungseinheit und eines Peripheriegerätes sowie die einzelnen Schritte für die Zuordnung von Patient, Peripheriegerät und Behandlungseinheit unter Bezugnahme auf Figur 3 beschrieben.

Das Peripheriegerät 5, beispielsweise ein Blutdruckmonitor, verfügt über verschiedene nur schematisch dargestellte Komponenten 6 zur Überwachung des Patienten, beispielsweise die Komponenten zur Überwachung des Blutdrucks. Zur Kommunikation mit der zugehörigen Behandlungseinheit 1 verfügt das Peripheriegerät 5 über Mittel 7 zum Senden von Steuersignalen an die Behandlungseinheit und Mittel 8 zum Senden von Patientensignalen an die Behandlungseinheit. Vorzugsweise erfolgt die Datenübertragung bidirektional, wobei die Datenübertragungsmittel sowohl Mittel zum Senden als auch Empfangen von Steuer- und Patientensignalen umfassen. Des weiteren weist das Peripheriegerät 5 Mittel 9 zur Überwachung der Zuordnung des Peripheriegerätes zu dem Patienten 2 und Mittel 10 zum Anmelden des Peripheriegerätes bei der zugehörigen Behandlungseinheit 1 auf.

Die Behandlungseinheit 1, beispielsweise eine Blutbehandlungsvorrichtung mit einem extrakorporalen Blutkreislauf, verfügt über verschiedene nur schematisch dargestellte Komponenten 11 zur Behandlung des Patienten 2, beispielsweise einen Dialysator, Pumpen etc. Der Patient 2 ist mit der Behandlungseinheit 1 fest verbunden, beispielsweise über eine venöse und arterielle Blutleitung 3, 4. Damit ist eine feste Zuordnung zwischen Patient und Behandlungseinheit gegeben.

Zur Kommunikation mit dem zugehörigen Peripheriegerät 5 verfügt die Behandlungseinheit 1 bei einer bidirektionalen Datenübertragung über Mittel 12 zum Senden und Empfangen von Steuersignalen und Mittel 13 zum Senden und Empfangen von Patientensignalen zu dem bzw. von dem Peripheriegerät. Des weiteren weist die Behandlungseinheit Mittel 14 zur Eingabe einer Bestätigung einer erfolgreichen Zuordnung von dem Peripheriegerät zur der Behandlungseinheit und Mittel 15 zur Überwachung der Zuordnung des Peripheriegerätes zu der Behandlungseinheit auf.

Die Datenübertragung zwischen Behandlungseinheit 1 und Peripheriegerät 5 erfolgt per Funk, wobei die hierzu erforderlichen Sender und Empfänger sowie Schnittstellen dem Fachmann allgemein bekannt sind. Das Peripheriegerät und die Behandlungseinheit erlauben vorzugsweise eine bidirektionale Datenübertragung, es ist aber auch möglich, dass die Übertragung der Daten, die sowohl Steuer- als auch Patientensignale umfassen, nur von dem Peripheriegerät auf die Behandlungseinheit erfolgt.

Nachfolgend wird die Funktion der Behandlungseinheiten und Peripheriegeräte im Einzelnen beschrieben.

Zunächst wählt das Bedienungspersonal ein Peripheriegerät 5, beispielsweise einen Blutdruckmonitor, aus den zur Verfügung stehenden Peripheriegeräten 5, 5' aus. Anschließend ordnet das Bedienungspersonal das ausgewählte Peripheriegerät einem bestimmten Patienten 2 zu. Die Zuordnung kann dadurch erfolgen, dass das Peripheriegerät an den Patienten angeschlossen wird. Dieser Vorgang wird mit den Mitteln 9 zur Überwachung der Zuordnung des Peripheriegerätes zu dem Patienten überwacht. Hierzu weisen die Mittel 9 zur Überwachung der Zuordnung Mittel 9a zur manuellen Eingabe, beispielsweise einen Schalter oder Taster auf. Dieser Schalter oder Taster wird von dem Bedienungspersonal betätigt, wenn das Peripheriegerät an den Patienten angeschlossen ist.

Anstelle der Mittel 9a zur manuellen Eingabe können auch Mittel 9b zur automatischen Erkennung des dem Patienten zugeordneten Peripheriegerätes vorgesehen sein. Es ist aber auch möglich, dass sowohl Mittel zur manuellen Eingabe 9a als auch zur automatischen Erkennung 9b vorgesehen sind. Die automatische Erkennung der Zuordnung kann beispielsweise über einen Nährungssensor erfolgen, der beim Anbringen des Peripheriegerätes die Zuordnung zum Patienten detektiert. Es ist aber auch möglich, die Zuordnung zu dem Patienten dadurch automatisch zu erkennen, dass eine Testroutine, die nur dann durchgeführt werden kann, wenn das Peripheriegerät an den Patienten angeschlossen ist, erfolgreich abgeschlossen ist.

Nach der erfolgreichen Zuordnung des Peripheriegerätes 5 zum Patienten 2 sendet das Peripheriegerät 5 ein Anmeldesignal an die zugehörige Behandlungseinheit 1. Daraufhin werden die Mittel 9a, 9b zur manuellen Eingabe oder automatischen Erkennung deaktiviert, so dass Fehleingaben ausgeschlossen sind.

Die Mittel 15 zur Überwachung der Zuordnung des Peripheriegerätes zu der Behandlungseinheit verfügen über ein Zeitglied 15A mit einer vorgegebenen Zeitkonstanten, beispielsweise 30 bis 60 Sekunden, welches mit der Registrierung des Anmeldesignals gestartet wird. Wenn innerhalb dieses Zeitfenster Anmeldeversuche anderer Peripheriegeräte erfolgen sollten, wird die Routine abgebrochen, so dass eine neue Anmeldung und Bestätigung erforderlich ist. Es ist aber auch möglich, dass die Behandlungseinheit 1 innerhalb dieses Zeitfensters den Anmeldeversuch eines anderen Peripheriegerätes 5' nicht mehr annimmt.

Die erfolgreiche Zuordnung von Peripheriegerät und Behandlungseinheit wird dem Bedienungspersonal angezeigt. Hierfür verfügt das Peripheriegerät 5 und/oder die Behandlungseinheit über eine optische und/oder akustische Anzeigeeinheit 16, 17. Das Bedienungspersonal muss nun innerhalb des Zeitfensters von 60 Sekunden überprüfen, ob die Zuordnung korrekt ist. Wenn dies der Fall ist, bestätigt das Bedienungspersonal die Zuordnung durch Betätigung der Mittel 14 zur Eingabe der Bestätigung an der Behandlungseinheit, beispielsweise durch Betätigung eines Schalters oder Tasters. Wenn hingegen keine manuelle Bestätigung der korrekten Zuordnung durch das Bedienungspersonal innerhalb dieses Zeitfensters erfolgt, wird der Vorgang abgebrochen, so dass eine neue Anmeldung erforderlich ist.

Obwohl nur eine drahtlose Verbindung zwischen Peripheriegerät 5 und Behandlungseinheit 1 besteht, die ein Anschließen des Peripheriegerätes an die Behandlungseinheit nicht erforderlich macht, kann das Bedienungspersonal die Zuordnung von Peripheriegerät und Behandlungseinheit dadurch aktiv nachvollziehen, dass an der Behandlungseinheit, die dem Peripheriegerät zugeordnet worden ist, die erfolgreiche Zuordnung bestätigt wird. Die manuelle Bestätigung der Zuordnung entspricht dem Einstecken eines Steckers in eine Buchse, was ansonsten bei einer konventionellen Verbindung der Geräte mit einem Anschlusskabel erforderlich ist.

Erst wenn die Zuordnung erfolgreich war und bestätigt worden ist, wird die Übertragung der Patientensignale, beispielsweise die Messwerte für den Blutdruck des Patienten, in Gang gesetzt, indem die Mittel 13 zum Empfangen von Patientensignalen von dem zugeordneten Peripheriegerät 5 für den eigentlichen Datentransfer aktiviert werden.

Zur Identifizierung der Patientensignale verfügen das Peripheriegerät 5 und die Behandlungseinheit 1 jeweils über einen Speicher 18, 19, wobei in dem Speicher 18 des Peripheriegerätes ein Identifikationssignal ID und in dem Speicher 19 der Behandlungseinheit ein dem jeweiligen Peripheriegerät zugeordnetes Referenzsignal nicht flüchtig gespeichert sind.

Im Zuge der Anmeldung des Peripheriegerätes bei der Behandlungseinheit sendet das Peripheriegerät zusammen mit dem Anmeldesignal das Identifikationssignal ID, das in dem Speicher 18 des Peripheriegerätes gespeichert ist. Die Behandlungseinheit vergleicht dann die in dem Speicher 19 gespeicherten Referenzsignale mit dem empfangenen Identifikationssignal ID des Peripheriegerätes. Stimmen Identifikationssignal ID und Referenzsignal überein, hat die Behandlungseinheit das zugehörige Peripheriegerät erkannt. Dadurch wird ausgeschlossen, dass die Behandlungseinheit die Patientensignale anderer Peripheriegeräte empfängt.

Neben der statischen Zuordnung, bei der in den Speichern 18, 19 des Peripheriegerätes 5 und der Behandlungseinheit 1 Identifikations- bzw. Referenzsignale gespeichert sind, die eine feste Zuordnung zwischen Peripheriegerät und Behandlungseinheit schaffen, ist auch eine dynamische Zuordnung möglich.

Bei der dynamischen Zuordnung wird das in dem Speicher 18 des Peripheriegerätes 5 nicht flüchtig gespeicherte Identifikationssignal ID bei der Anmeldung zusammen mit dem Anmeldesignal an die Behandlungseinheit gesendet, welches die Behandlungseinheit in dem Speicher 19 speichert. Nach der Bestätigung der erfolgreichen Zuordnung von Peripheriegerät und Behandlungseinheit sendet die Behandlungseinheit ein in dem Speicher 19 gespeichertes Identifikationssignal ID der Behandlungseinheit an das Peripheriegerät. Während der nachfolgenden Datenübertragung werden dann beide Identifikationssignale benutzt, wobei mit der Bestätigung der Anmeldung des Peripheriegerätes bei der Behandlungseinheit durch das Bedienungspersonal eine falsche Zuordnung von Peripheriegerät und Behandlungseinheit verhindert wird.

## Patentansprüche

1. Anordnung aus einer Mehrzahl von medizinischen Behandlungseinheiten (1, 1'), die jeweils einem Patienten zuzuordnen sind, und einer Mehrzahl von Peripheriegeräten (5, 5'), die jeweils einem Patienten zuzuordnen sind, wobei
die Peripheriegeräte aufweisen:
Mittel (7) zum Senden von Steuersignalen an eine Behandlungseinheit (1),
Mittel (8) zum Senden von Patientensignalen an eine Behandlungseinheit (1), und
die Behandlungseinheiten (1) aufweisen:
Mittel (12) zum Empfangen von Steuersignalen von einem Peripheriegerät (5),
Mittel (13) zum Empfangen von Patientensignalen von einem Peripheriegerät (5),
**dadurch gekennzeichnet, dass**
die Peripheriegeräte aufweisen:
Mittel (9) zur Überwachung der Zuordnung eines Peripheriegerätes zu einem Patienten, und
Mittel (10) zum Anmelden des Peripheriegerätes bei einer Behandlungseinheit die derart mit den Mitteln (9) zur Überwachung der Zuordnung eines Periphenegerätes zu einem Patienten und den Mitteln (7) zum Senden von Steuersignalen zusammenwirken, dass bei erfolgreicher Zuordnung des Peripheriegerätes zu einem Patienten ein Anmeldesignal an die dem Patienten zugeordnete Behandlungseinheit gesendet wird, und
die Behandlungseinheiten (1) aufweisen:
Mittel (14) zur Eingabe einer Bestätigung einer erfolgreichen Zuordnung von einem Peripheriegerät (5) zu einer Behandlungseinheit (1), und
Mittel (15) zur Überwachung der Zuordnung eines Peripheriegerätes zu der Behandlungseinheit, die derart mit den Mitteln (12) zum Empfangen von Steuersignalen, den Mitteln (14) zur Eingabe einer Bestätigung und den Mitteln (13) zum Empfangen von Patientensignalen zusammenwirken, dass nach dem Empfang eines Anmeldesignals von einem Peripherie gerät überprüft wird, ob eine erfolgreiche Zuordnung von einem Peripheriegerät zu einer Behandlungseinheit bestätigt worden ist, und die Mittel (13) zum Empfangen von Patientensignalen von dem zugeordneten Peripheriegerät aktiviert werden, wenn eine erfolgreiche Zuordnung von einem Peripheriegerät zu einer Behandlungseinheit bestätigt worden ist.

2. Anordnung nach Anspruch 1, **dadurch gekennzeichnet, dass** die Mittel (15) zur Überwachung der Zuordnung eines Peripheriegerätes (5) zu einer Behandlungseinheit (1) ein Zeitglied (15A) mit einer vorgegebenen Zeitkonstante aufweisen, das nach dem Empfang des Anmeldesignals gestartet wird, und dass die Mittel (15) zur Überwachung der Zuordnung derart ausgebildet sind, dass innerhalb der vorgegebenen Zeitkonstante überprüft wird, ob eine erfolgreiche Zuordnung von einem Peripheriegerät zu einer Behandlungseinheit bestätig worden ist.

3. Anordnung nach Anspruch 2, **dadurch gekennzeichnet, dass** die Mittel (15) zur Überwachung der Zuordnung eines Peripheriegerätes (5) zu einer Behandlungseinheit (1) derart ausgebildet sind, dass die Mittel (13) zum Empfangen von Patientensignalen von einem Peripheriegeräte nicht aktiviert werden, wenn innerhalb der vorgegebenen Zeitkonstante Anmeldesignale von mehreren Peripheriegeräten empfangen worden sind.

4. Anordnung nach einem der Ansprüche 1 bis 3, **dadurch gekennzeichnet, dass** die Mittel (15) zur Überwachung der Zuordnung eines Peripheriegerätes (5) zu einer Behandlungseinheit (1) Mittel zum Vergleichen eines Identifikationssignals eines Peripheriegerätes mit einem dem Peripheriegerät zugewiesenen Referenzsignal der Behandlungseinheit aufweisen, die mit den Mitteln (13) zum Empfangen der Patientensignale derart zusammenwirken, dass bei einer Übereinstimmung von Identifikationssignal und Referenzsignal die Mittel (13) zum Empfangen von Patientensignalen aktiviert werden.

5. Anordnung nach einem der Ansprüche 1 bis 4, **dadurch gekennzeichnet, dass** das Peripheriegerät (5) Mittel (18) zum Speichern, eines Identifikationssignals aufweist.

6. Anordnung nach Anspruch 5, **dadurch gekennzeichnet, dass** die Mittel (10) zum Anmelden eines Peripheriegerätes (5) bei einer Behandlungseinheit (1) derart mit den Mitteln (7) zum Senden von Steuersignalen zusammenwirken, dass das Identifikationssignal des Peripheriegerätes (5) zusammen mit dem Anmeldesignal an die Behandlungseinheit (1) gesendet wird.

7. Anordnung nach einem der Ansprüche 1 bis 6, **dadurch gekennzeichnet, dass** die Behandlungseinheit (1) Mittel (19) zum Speichern eines Identiitkationssignals aufweist.

8. Anordnung nach einem der Ansprüche 1 bis 7, **dadurch gekennzeichnet, dass** die Mittel (9) zur Überwachung der Zuordnung eines Peripheriegerätes (5) zu einem Patienten Mittel (9a) zur manuellen Eingabe aufweisen.

9. Anordnung nach einem der Ansprüche 1 bis 7, **dadurch gekennzeichnet, dass** die Mittel (9) zur Überwachung der Zuordnung eines Peripheriegerätes (5) zu einem Patienten Mittel (9b) zur automatischen Erkennung eines dem Patienten zugeordneten Peripheriegerätes aufweisen.

10. Anordnung nach einem der Ansprüche 1 bis 9, **dadurch gekennzeichnet, dass** die Mittel (9) zur Überwachung der Zuordnung eines Peripheriegerätes (5) zu einem Patienten und/oder die Mittel (14) zur Eingabe einer Bestätigung der Zuordnung eines Peripheriegerätes zu einer Behandlungseinheü Mittel (16, 17) zur Anzeige der Zuordnung von Peripheriegerät und Behandluagseinheit aufweisen.

11. Anordnung nach einem der Ansprüche 1 bis 10, **dadurch gekennzeichnet, dass** die Mittel (7, 8) zum Senden von Steuer- und Patientensignalen des Peripheriegerätes (5) und die Mittel (12, 13) zum Empfangen von Steuer- und Patientensignalen der Behandlungseinheit (1) Mittel zur drahtlosen Übertragung der Signale sind.

12. Anordnung nach einem der Ansprüche 1 bis 11, **dadurch gekennzeichnet, dass** die Behandlungseinheit (1) eine Blutbehandlungsvorrichtung mit einem extrakorporalen Blutkreislauf ist.

## Claims

1. An arrangement comprising a plurality of medical treatment units (1, 1'), which are each to be allocated to a patient, and a plurality of peripheral devices (5, 5'), which are each to be allocated to a patient, wherein
the peripheral devices comprise:
means (7) for sending control signals to a treatment unit (1),
means (8) for sending patient signals to a treatment unit (1), and
the treatment units (1) comprise:
means (12) for receiving control signals from a peripheral device (5),
means (13) for receiving patient signals from a peripheral device (5),
**characterised in that**
the peripheral devices comprise:
means (9) for monitoring the allocation of a peripheral device to a patient, and
means (10) for logging on the peripheral device with a treatment unit, said means cooperating with the means (9) for monitoring the allocation of a peripheral device to a patient and the means (7) for sending control signals in such a way that, when a successful allocation of the peripheral device to a patient has taken place, a log-on signal is sent to the treatment unit allocated to the patient, and
the treatment units (1) comprise:
means (14) for inputting an acknowledgement of a successful allocation of a peripheral device (5) to a treatment unit (1), and
means (15) for monitoring the allocation of a peripheral device to the treatment unit, said means cooperating with the means (12) for receiving control signals, the means (14) for inputting an acknowledgement and the means (13) for receiving patient signals in such a way that, after a log-on signal has been received from a peripheral device, a check is made to establish whether a successful allocation of a peripheral device to a treatment unit has been acknowledged, and the means (13) for receiving patient signals from the allocated peripheral device are activated when a successful allocation of a peripheral device to a treatment unit has been acknowledged.

2. The arrangement according to claim 1, **characterised in that** the means (15) for monitoring the allocation of a peripheral device (5) to a treatment unit (1) comprise a timing element (15A) with a preset timing constant which is started after the log-on signal has been received, and **in that** the means (15) for monitoring the allocation are designed in such a way that, within the preset timing constant, a check is made to establish whether a successful allocation of a peripheral device to a treatment unit has been acknowledged.

3. The arrangement according to claim 2, **characterised in that** the means (15) for monitoring the allocation of a peripheral device (5) to a treatment unit (1) are designed in such a way that the means (13) for receiving patient signals from a peripheral device are not activated if log-on signals have been received from a plurality of peripheral devices within the preset timing constant.

4. The arrangement according to any one of claims 1 to 3, **characterised in that** the means (15) for monitoring the allocation of a peripheral device (5) to a treatment unit (1) comprise means for comparing an identification signal of a peripheral device with a reference signal of the treatment unit allocated to the peripheral device, said means cooperating with the means (13) for receiving the patient signals in such a way that the means (13) for receiving patient signals are activated when there is agreement of identification signal and reference signal.

5. The arrangement according to any one of claims 1 to 4, **characterised in that** the peripheral device (5) comprises means (18) for storing an identification signal.

6. The arrangement according to claim 5, **characterised in that** the means (10) for logging on a peripheral device (5) with a treatment unit (1) cooperate with the means (7) for sending control signals, in such a way that the identification signal of the peripheral device (5) is sent together with the log-on signal to the treatment unit (1).

7. The arrangement according to any one of claims 1 to 6, **characterised in that** the treatment unit (1) comprises means (19) for storing an identification signal.

8. The arrangement according to any one of claims 1 to 7, **characterised in that** the means (9) for monitoring the allocation of a peripheral device (5) to a patient comprise means (9a) for a manual input.

9. The arrangement according to any one of claims 1 to 7, **characterised in that** the means (9) for monitoring the allocation of a peripheral device (5) to a patient comprise means (9b) for the automatic recognition of a peripheral device allocated to the patient.

10. The arrangement according to any one of claims 1 to 9, **characterised in that** the means (9) for monitoring the allocation of a peripheral device (5) to a patient and/or the means (14) for inputting an acknowledgement of the allocation of a peripheral device to a treatment unit comprise means (16, 17) for displaying the allocation of peripheral device and treatment unit.

11. The arrangement according to any one of claims 1 to 10, **characterised in that** the means (7, 8) for sending control signals and patient signals of the peripheral device (5) and the means (12, 13) for receiving control signals and patient signals of the treatment unit (1) are means for the wireless transmission of the signals.

12. The arrangement according to any one of claims 1 to 11, **characterised in that** the treatment unit (1) is a blood treatment apparatus with an extracorporeal blood circuit.

## Revendications

1. Ensemble constitué d'une pluralité d'unités de traitement (1, 1') médicales, lesquelles sont à affecter respectivement à un patient, et d'une pluralité d'appareils périphériques (5, 5'), qui sont à affecter respectivement à un patient, sachant que
les appareils périphériques présentent :
des moyens (7) servant à envoyer des signaux de commande à une unité de traitement (1),
des moyens (8) servant à envoyer des signaux relatifs à un patient à une unité de traitement (1), et
sachant que les unités de traitement (1) présentent :
des moyens (12) servant à recevoir des signaux de commande en provenance d'un appareil périphérique (5),
des moyens (13) servant à recevoir des signaux relatifs à un patient en provenance d'un appareil périphérique (5),
**caractérisé en ce que**
les appareils périphériques présentent :
des moyens (9) servant à surveiller l'affectation d'un appareil périphérique à un patient, et
des moyens (10) servant à notifier l'appareil périphérique à une unité de traitement, lesquels coopèrent avec les moyens (9) servant à surveiller l'affectation d'un appareil périphérique à un patient et avec les moyens (7) servant à envoyer des signaux de commande de telle manière que lorsque l'appareil périphérique est affecté avec succès à un patient, un signal de notification est envoyé à l'unité de traitement affectée au patient, et
**en ce que** les unités de traitement (1) présentent :
des moyens (14) servant à saisir une confirmation de l'affectation réalisée avec succès d'un appareil périphérique (5) à une unité de traitement (1), et
des moyens (15) servant à surveiller l'affectation d'un appareil périphérique à l'unité de traitement, lesquels coopèrent avec les moyens (12) servant à recevoir des signaux de commande, avec les moyens (14) servant à saisir une confirmation et avec les moyens (13) servant à recevoir des signaux relatifs à un patient de telle manière qu'on vérifie à réception d'un signal de notification en provenance d'un appareil périphérique si une affectation réalisée avec succès d'un appareil périphérique à une unité de traitement a été confirmée, et que les moyens (13) servant à recevoir des signaux relatifs à un patient en provenance de l'appareil périphérique sont activés lorsqu'une affectation réalisée avec succès d'un appareil périphérique à une unité de traitement a été confirmée.

2. Ensemble selon la revendication 1, **caractérisé en ce que** les moyens (15) servant à surveiller l'affectation d'un appareil périphérique (5) à une unité de traitement (1) présentent un organe de temps (15A) doté d'une constante de temps prédéfinie, lequel est activé à réception du signal de notification, et **en ce que** les moyens (15) servant à surveiller l'affectation sont réalisés de telle manière qu'on vérifie dans le cadre de la constante de temps prédéfinie si une affectation réalisée avec succès d'un appareil périphérique à une unité de traitement a été confirmée.

3. Ensemble selon la revendication 2, **caractérisé en ce que** les moyens (15) servant à surveiller l'affectation d'un appareil périphérique (5) à une unité de traitement (1) sont réalisés de telle manière que les moyens (13) servant à recevoir des signaux relatifs à un patient en provenance d'un appareil périphérique ne sont pas activés lorsque dans le cadre de la constante de temps prédéfinie, des signaux de notification en provenance de plusieurs appareils périphériques ont été reçus.

4. Ensemble selon l'une quelconque des revendications 1 à 3, **caractérisé en ce que** les moyens (15) servant à surveiller l'affectation d'un appareil périphérique (5) à une unité de traitement (1) présentent des moyens servant à comparer un signal d'identification d'un appareil périphérique à un signal de référence, attribué à l'appareil périphérique, de l'unité de traitement, lesquels moyens coopèrent avec les moyens (13) servant à recevoir les signaux relatifs à un patient de telle manière qu'en cas de concordance entre le signal d'identification et le signal de référence, les moyens (13) servant à recevoir des signaux relatifs à un patient sont activés.

5. Ensemble selon l'une quelconque des revendications 1 à 4, **caractérisé en ce que** l'appareil périphérique (5) présente des moyens (18) servant à mémoriser un signal d'identification.

6. Ensemble selon la revendication 5, **caractérisé en ce que** les moyens (10) servant à notifier un appareil périphérique (5) auprès d'une unité de traitement (1) coopèrent avec les moyens (7) servant à envoyer des signaux de commande de telle manière que le signal d'identification de l'appareil périphérique (5) est envoyé à l'unité de traitement (1) conjointement avec le signal de notification.

7. Ensemble selon l'une quelconque des revendications 1 à 6, **caractérisé en ce que** l'unité de traitement (1) présente des moyens (19) servant à mémoriser un signal d'identification.

8. Ensemble selon l'une quelconque des revendications 1 à 7, **caractérisé en ce que** les moyens (9) servant à surveiller l'affectation d'un appareil périphérique (5) à un patient présentent des moyens (9a) servant à la saisie manuelle.

9. Ensemble selon l'une quelconque des revendications précédentes 1 à 7, **caractérisé en ce que** les moyens (9) servant à surveiller l'affectation d'un appareil périphérique (5) à un patient présentent des moyens (9b) servant à identifier de manière automatique un appareil périphérique affecté au patient.

10. Ensemble selon l'une quelconque des revendications 1 à 9, **caractérisé en ce que** les moyens (9) servant à surveiller l'affectation d'un appareil périphérique (5) à un patient et/ou les moyens (14) servant à saisir une confirmation de l'affectation d'un appareil périphérique à une unité de traitement présentent des moyens (16, 17) servant à afficher l'affectation de l'appareil périphérique et de l'unité de traitement.

11. Ensemble selon l'une quelconque des revendications 1 à 10, **caractérisé en ce que** les moyens (7, 8) de l'appareil périphérique (5) servant à envoyer des signaux de commande et des signaux relatifs à un patient et les moyens (12, 13) de l'unité de traitement (1) servant à recevoir des signaux de commande et des signaux relatifs à un patient sont des moyens destinés à la transmission sans fil de signaux.

12. Ensemble selon l'une quelconque des revendications 1 à 11, **caractérisé en ce que** l'unité de traitement (1) est un dispositif de traitement du sang équipé d'un circuit sanguin extracorporel.
